# EUROPÄISCHE PATENTANMELDUNG

(11) **EP 1 905 471 A2**
(43) Veröffentlichungstag der Anmeldung: **02.04.2008**
(21) Anmeldenummer: 07018055.9
(22) Anmeldetag: 14.09.2007
(51) Int. Cl.: A61M 15/00, A61M 15/08

(54) **Inhalationsvorrichtung**

(30) Priorität: 28.09.2006 DE 102006047668
(71) Anmelder: Ing. Erich Pfeiffer GmbH, 78315 Radolfzell (DE)
(72) Erfinder: Carrico, Silvio, 78224 Singen (DE)
(74) Vertreter: Patentanwälte Ruff, Wilhelm, Beier, Dauster & Partner

(57) **Zusammenfassung**

Inhalationsvorrichtung für ein insbesondere pulverförmiges Medium (30) mit einem Gehäuse (10) mit mindestens einem Medienspeicher (24) und einem Auslasskanal (42), wobei ein erstes Ende (42a) des Auslasskanals (42) im Bereich des Medienspeichers (24) mündet und ein zweites Ende (42b) zum bündigen Ansetzen an eine Atemöffnung eines Benutzers ausgebildet ist, so dass das im Medienspeicher (24) gelagerte Medium (30) durch Einatmen seitens des Benutzers inhalierbar ist, und wobei eine Atmungsunterstützungsvorrichtung (60) vorgesehen ist, die lösbar mit dem Gehäuse (10) verbindbar ist und aus der dem Gehäuse (10) ein die Inhalation unterstützender Luftstrom mittels einer manuellen Betätigung zuführbar ist.

Verwendung für die Applikation von Medikamenten.

## Beschreibung

Die Erfindung betrifft eine Inhalationsvorrichtung für ein insbesondere pulverförmiges Medium mit einem Gehäuse mit mindestens einen Medienspeicher und einem Auslasskanal, wobei ein erstes Ende des Auslasskanals im Bereich des Medienspeichers mündet und ein zweites Ende zum bündigen Ansetzen an eine Atemöffnung eines Benutzers ausgebildet ist, so dass das im Medienspeicher gelagerte Medium durch Einatmen seitens des Benutzers inhalierbar ist.

Eine derartige Inhalationsvorrichtung ist beispielsweise aus der EP 0957962 B1 bekannt. Die dort offenbarte Vorrichtung besteht aus einem Kunststoffgehäuse mit zwei Medienspeichern, deren pulverförmiger Inhalt durch Einführen eines Ansatzstückes in ein Nasenloch des Benutzers und anschließendem Einatmen durch das Ansatzstück hindurch ausgetragen wird. Die Vorrichtung eignet sich insbesondere für nasal einzunehmende Medien, die eine medikamentöse Wirkstoffkombination beinhalten.

Als nachteilig an dieser und ähnlichen Vorrichtungen aus dem Stand der Technik wird angesehen, dass insbesondere besonders junge und ältere Benutzer Probleme damit haben können, den erforderlichen Einatmungsvorgang so kräftig und lang durchzuführen, wie es für eine Inhalation des gesamten Mediums erforderlich ist.

### Aufgabe und Lösung

Die Aufgabe der Erfindung besteht darin, eine gattungsgemäße Inhalationsvorrichtung zu schaffen, deren Austragvorgang auch dann erfolgreich und vollständig durchgeführt werden kann, wenn ungeübte oder körperlich eingeschränkte Benutzer, insbesondere jüngere oder ältere Benutzer, die Inhalationsvorrichtung verwenden.

Erfindungsgemäß wird diese Aufgabe durch eine Inhalationsvorrichtung der eingangs genannten Art gelöst, welche eine Atmungsunterstützungsvorrichtung aufweist, die lösbar mit dem Gehäuse verbindbar ist und aus der dem Gehäuse ein die Inhalation unterstützender Luftstrom mittels einer manuellen Betätigung zuführbar ist.

Es handelt sich bei der erfindungsgemäßen Vorrichtung um ein zweiteiliges System, dessen Komponenten durch den Benutzer montierbar und demontierbar sind. Die eine Komponente, das Gehäuse der Inhalationseinrichtung, ist dabei vorzugsweise auch separat verwendbar, so dass die Atmungsunterstützungsvorrichtung nur von den Benutzern verwendet zu werden braucht, denen ein bestimmungsgerechter Inhalationsvorgang ansonsten nicht möglich wäre. Das Gehäuse der Inhalationsvorrichtung kann einteilig oder mehrteilig ausgeführt sein und ist vorzugsweise aus Kunststoff, beispielsweise im Spritzgussverfahren gefertigt. Es enthält den Medienspeicher, in dem das Medium bis zu einem Austragvorgang gelagert ist. Dieser Medienspeicher ist vorzugsweise bis zur Verwendung der Inhalationsvorrichtung mittels einer Folie verschlossen, die erst im Zuge einer Aktivierung für den Austragsvorgang zumindest partiell entfernt bzw. durchbrochen wird und die zuvor ein versehentliches Entweichen des Mediums verhindert. Der am Gehäuse vorgesehene Auslasskanal ist so gestaltet, dass er mit der Atemöffnung des Benutzers bündig abschließen kann. Bei einer erfindungsgemäßen Inhalationsvorrichtung für eine nasale Anwendung weist er vorzugsweise einen runden oder ovalen Querschnitt auf, dessen Durchmesser in etwa an die Größe eines Nasenlochs der betreffenden Zielgruppe angepasst ist.

Mit diesem Gehäuse der Inhalationsvorrichtung ist die Atmungsunterstützungsvorrichtung derart verbindbar, dass durch eine dafür vorgesehene Auslassöffnung der Atmungsunterstützungsvorrichtung austretende Luft in das Gehäuse einströmen kann. Das Gehäuse ist dabei so ausgebildet, dass dieser Luftstrom dem Medienspeicher zugeführt wird und dadurch das Medium in den Auslasskanal eingeblasen und dann ausgetragen wird. Die Erzeugung des Luftstroms kann unmittelbar durch die manuelle Betätigung erfolgen, beispielsweise unter Anwendung einer manuell betätigbaren Kolbenpumpe. Zweckmäßig kann jedoch auch die Verwendung eines aus einem Energiespeicher gespeisten Pumpmittels sein, beispielsweise eines elektrisch betriebenen, bei dem eine mittelbare manuelle Betätigung lediglich in Form einer Betätigung eines Schalters erfolgt. Die manuelle Betätigung erfolgt vorzugsweise an der Atmungsunterstützungsvorrichtung selbst oder im Zusammenspiel mit der Atmungsunterstützungsvorrichtung, beispielsweise durch Zusammendrücken des Verbundes aus Atmungsunterstützungsvorrichtung und Gehäuse. Denkbar sind jedoch auch Ausführungsformen, bei denen die Betätigung der Atmungsunterstützungsvorrichtung am Gehäuse erfolgt.

Bei der Anwendung einer erfindungsgemäßen Inhalationsvorrichtung werden der Einatmungsvorgang des Benutzers und die Betätigung der Atmungsunterstützungsvorrichtung vom Benutzer zeitlich so koordiniert, dass das pulverförmige Medium gleichzeitig durch das Einatmen des Benutzers in den Auslasskanal eingezogen und durch die Atmungsunterstützungsvorrichtung in den Auslasskanal eingeblasen wird. Der Inhalationsvorgang ist daher besonders sicher, und es ist auch bei problematischen Bedingungen, wie ungeübten oder körperlich eingeschränkten Benutzern oder auch besonders großen Mengen des Mediums, gewährleistet, dass die gesamte Medienmenge vollständig inhaliert wird.

Neben dem Vorteil in Hinblick auf eine erfolgreiche Inhalation auch durch problematische Zielgruppen, weist eine erfindungsgemäße Inhalationsvorrichtung noch weitere Vorteile auf. Die Atmungsunterstützungsvorrichtung kann mehrfach verwendet werden, was unter Umweltschutzgesichtspunkten von Vorteil ist. Des Weiteren kann bei entsprechender Gestaltung das Gehäuse ohne spezielle Anpassungen mit und ohne Atmungsunterstützungsvorrichtung verwendet werden, so dass gegenüber alternativen Ausführungsformen, bei denen die Atmungsunterstützungsvorrichtung integraler Bestandteil der Inhalationsvorrichtung ist, größere und damit wirtschaftlichere Stückzahlen des Gehäuses produziert werden können.

In einer Weiterbildung der Erfindung ist die Atmungsunterstützungsvorrichtung für eine manuelle Betätigung in einer Betätigungsrichtung ausgebildet, die zu einer Haupterstreckungsrichtung des Auslasskanals parallel ist.

Durch diese Form der Gestaltung wird die Gefahr vermieden, dass im Zuge der manuellen Betätigung der Atmungsunterstützungsvorrichtung ein Herausrutschen oder Abrutschen des Auslasskanals aus der Atemöffnung des Benutzers verursacht wird. Eine Betätigung führt allenfalls zu einer Bewegung in der Haupterstreckungsrichtung des Auslasskanals, bei einer nasalen Anwendung also dazu, dass der Auslasskanal in das Nasenloch des Benutzers etwas weiter hineingedrückt wird.

In einer Weiterbildung der Erfindung weißt die Atmungsunterstützungsvorrichtung eine Kompressionskammer auf, deren Volumen durch die manuelle Betätigung unmittelbar mechanisch reduzierbar ist.

Eine solche Atmungsunterstützungsvorrichtung kann besonders einfach hergestellt werden und ist dadurch besonders preisgünstig. Im einfachsten Fall weist die Atmungsunterstützungsvorrichtung einen einfachen Pumpball auf, der zur Betätigung mit der ganzen Hand umschlossen und dann zusammengedrückt wird.

Besonders vorteilhaft ist es, wenn die Atmungsunterstützungsvorrichtung einen Faltenbalg aufweist.

Ein solcher die Kompressionskammer bildender Faltenbalg kann auf Grund der konstruktiv bedingten definierten Betätigungsrichtung mit nur einem oder zwei Fingern bedient werden, so dass die gesamte Inhalationsvorrichtung mit nur einer Hand gehandhabt werden kann. Ein solcher Faltenbalg ist vorzugsweise aus einem Elastomer oder einem Thermoplast, beispielsweise aus Polyethylen, gefertigt. Vorzugsweise ist der Faltenbalg einstückig mit einem sich daran anschließenden Anschlussabschnitt zur Befestigung am Gehäuse ausgebildet, wobei das elastische Material bei der Befestigung der Atmungsunterstützungsvorrichtung am Gehäuse vorteilhaft ist. Es sind jedoch auch Ausführungsformen zweckmäßig, bei denen der Anschlussabschnitt als getrenntes Bauteil vorgesehen ist, welches beispielsweise über ein formschlüssiges Eingreifen eines umlaufenden Stegs des Faltenbalgs in eine umlaufende Nut des Anschlussabschnitts mit dem Faltenbalg verbunden ist. Die Orientierung des Faltenbalgs ist vorzugsweise dergestalt, dass eine Faltenbalgkompression in Richtung auf das Gehäuse der Atmungsunterstützungsvorrichtung zu erfolgt. Es sind aber auch andere Ausführungsformen denkbar, beispielsweise solche, bei denen der Faltenbalg durch Zusammendrücken seiner beiden Stirnseiten betätigt wird, wobei das Gehäuse an einer dazwischenliegenden Seite des Faltenbalgs vorgesehen ist. Der Faltenbalg ist vorzugsweise mittels eines Spritzblasverfahrens oder eines Spritzstreckblasverfahrens hergestellt.

In einer Weiterbildung der Erfindung ist an einer der Atmungsunterstützungsvorrichtung gegenüberliegenden Seite des Gehäuses einer Fingerauflage vorgesehen.

Diese Fingerauflage gewährleistet eine sichere Handhabung, bei der die Betätigung der Inhalationsvorrichtung, insbesondere bei Verwendung einer Atmungsunterstützungsvorrichtung mit einer definierten Betätigungsrichtung, durch einfaches Zusammendrücken der an gegenüberliegenden Seiten der Inhalationsvorrichtung angreifenden Finger durchgeführt wird.

In einer Weiterbildung der Erfindung sind die Atmungsunterstützungsvorrichtung und das Gehäuse in einem Kontaktbereich derart ausgebildet, dass eine durch die manuelle Betätigung aufgebrachte Kraft zu einem dichten Aufeinanderpressen von gehäuseseitigen und atmungsunterstützungsvorrichtungsseitigen Abschnitten im Kontaktbereich führt.

Der Kontaktbereich kann beispielsweise durch ein umlaufende ringförmige Fläche am Gehäuse der Inhalationsvorrichtung sowie eine damit korrespondierende umlaufende Fläche an der Atmungsunterstützungsvorrichtung gebildet sein. Wenn zum Zweck der Betätigung eine Kraft ausgeübt wird, durch die das Gehäuse und die Atmungsunterstützungsvorrichtung aufeinander zu gedrückt werden, so führt dies zu einer besonders sicheren Anlage der Flächen aneinander. Ein Austreten relevanter Mengen der aus der Atmungsunterstützungsvorrichtung ausströmenden Luft in die Umgebung wird dadurch unterbunden.

In einer Weiterbildung der Erfindung weisen die Atmungsunterstützungsvorrichtung und das Gehäuse in einem Kontaktbereich kraft-und/oder formschlüssig wirkende Dicht- und/oder Sicherungsmittel auf.

Diese Dicht- und/oder Sicherungsmittel gewährleisten eine sichere Verbindung des Gehäuses der Inhalationsvorrichtung mit der Atmungsunterstützungsvorrichtung. Durch sie ist gewährleistet, dass auch bei einem Überdruck in der Atmungsunterstützungsvorrichtung oder bei elastischer Verformung der Atmungsunterstützungsvorrichtung im Zuge der Betätigung ein Ablösen dieser vom Gehäuse vermieden wird. Als Dicht-und/oder Sicherungsmittel bieten sich insbesondere formschlüssige Mittel wie Rastanordnungen an, beispielsweise eine gehäuseseitige Rastlippe, die in eine atmungsunterstützungsvorrichtungsseitige Nut eingreift.

In einer Weiterbildung der Erfindung weisen die Sicherungsmittel einen gehäuseseitigen Aufschubkonus und atmungsunterstützungsvorrichtungsseitigen Aufschubabschnitt auf.

Ein derartiges Sicherungsmittel wirkt kraftschlüssig. Der Aufschubabschnitt wird auf den Aufschubkonus soweit aufgeschoben, bis durch eine dabei auftretende elastische Dehnung des Aufschubabschitts bzw. eine elastische Stauchung des Aufschubkonus eine ausreichende Haltekraft vorliegt. Besonders vorteilhaft sind dabei Ausgestaltungen, bei denen im Kontaktbereich ein Luftkanal zum Lufttransport in das Gehäuse offen bleibt, beispielsweise durch konusseitige Axialnuten oder durch aufschubabschnittsseitig innen angeformte Spannrippen, die im befestigten Zustand am Aufschubkonus anliegen, während Bereiche zwischen den Spannrippen vom Aufschubkonus beabstandet sind.

In einer Weiterbildung der Erfindung ist das Gehäuse mehrteilig, insbesondere zweiteilig ausgebildet, wobei in einem ersten Gehäuseteil der mindestens eine Medienspeicher ausgebildet ist und an einem zweiten Gehäuseteil der Auslasskanal ausgebildet ist und wobei ein umlaufender Kontaktbereich zwischen der Atmungsunterstützungsvorrichtung und dem zweiten Gehäuseteil als im Betrieb nach außen luftdichter Kontaktbereich ausgebildet ist.

Bei einer solchen Ausführungsform ist das Gesamtsystem aus Gehäuse und Atmungsunterstützungsvorrichtung im umlaufenden Kontaktbereich leicht abdichtbar. Die aus der Atmungsunterstützungsvorrichtung im Betrieb herausströmende Luft kann dabei durch Spaltöffnungen, die zwischen dem ersten und dem zweiten Gehäuseteil vorgesehen sind, vorbeiströmen und so auf die der Atmungsunterstützungsvorrichtung abgewandte Seite des ersten Gehäuseteils gelangen, auf der der mindestens eine Medienspeicher vorzugsweise angeordnet ist. Besonders bevorzugt sind Ausführungsformen, bei denen die beiden Gehäuseteile in Haupterstreckungsrichtung des Auslasskanals gegeneinander verschiebbar ausgebildet sind und bei denen eine Relativverschiebung zueinander im Zuge einer Aktivierung erfolgt, durch die eine das Medium nach außen abdeckende Schutzfolie geöffnet wird.

In einer Weiterbildung der Erfindung sind am Gehäuse lösbare Aktivierungssperrmittel vorgesehen, die bei der Zusammenfügung des Gehäuses mit der Atmungsunterstützungsvorrichtung verhindern, dass sich das erste Gehäuseteil und das zweite Gehäuseteil relativ zueinander verschieben.

Diese Aktivierungssperrmittel gewährleisten, dass das Aufschieben und Sichern der Atmungsunterstützungsvorrichtung auf das Gehäuse nicht zu einer Öffnung des vorzugsweise mittels einer Folie verschlossenen Medienspeichers führt. Dadurch ist es möglich, gehäuseseitig oder atmungsunterstützungsvorrichtungsseitig Sicherungsmittel vorzusehen, die erst durch Aufbringen einer erheblichen Kraft in einen gesicherten Zustand überführt werden, ohne das es dadurch zu einem versehentlichen Austragen des Mediums kommt. Solche Aktivierungssperrmittel können beispielsweise durch entfernbare umlaufende Sicherheitsstreifen zwischen den Gehäuseteilen, durch Sicherheitsstege zum Herausbrechen oder auch durch Bajonettmechanismen realisiert sein.

Bei einer Weiterbildung der Erfindung ist das Gehäuse im Spritzgussverfahren und/oder der Faltenbalg im Spritzblasverfahren hergestellt ist.

Diese Herstellungsverfahren stellen jeweils besonders preisgünstige und zuverlässige Herstellungsverfahren da.

### Kurzbeschreibung der Zeichnungen

Weitere Vorteile und Merkmale der Erfindung ergeben sich aus den Ansprüchen sowie der nachfolgenden Beschreibung eines bevorzugten Ausführungsbeispiels der Erfindung, das anhand der Zeichnungen dargestellt ist. Dabei zeigt:
- Figur 1: ein Inhalatorgehäuse in einem nicht aktivierten Zustand ohne Atmungsunterstützungsvorrichtung,
- Figur 2a und 2b: eine Atmungsunterstützungsvorrichtung mit einem Faltenbalg in einer Vorderansicht und einer Draufsicht,
- Figur 3: die aus dem Gehäuse der Figur 1 und der Atmungs-unterstützungsvorrichtung der Figuren 2a und 2b zusammengesetzte vollständige Inhalationsvorrichtung,
- Figur 4: die Inhalationsvorrichtung in einem aktivierten, betriebsfertigen Zustand und
- Figur 5: die Inhalationsvorrichtung während des Austragsvorgangs.

### Detaillierte Beschreibung der Ausführungsbeispiele

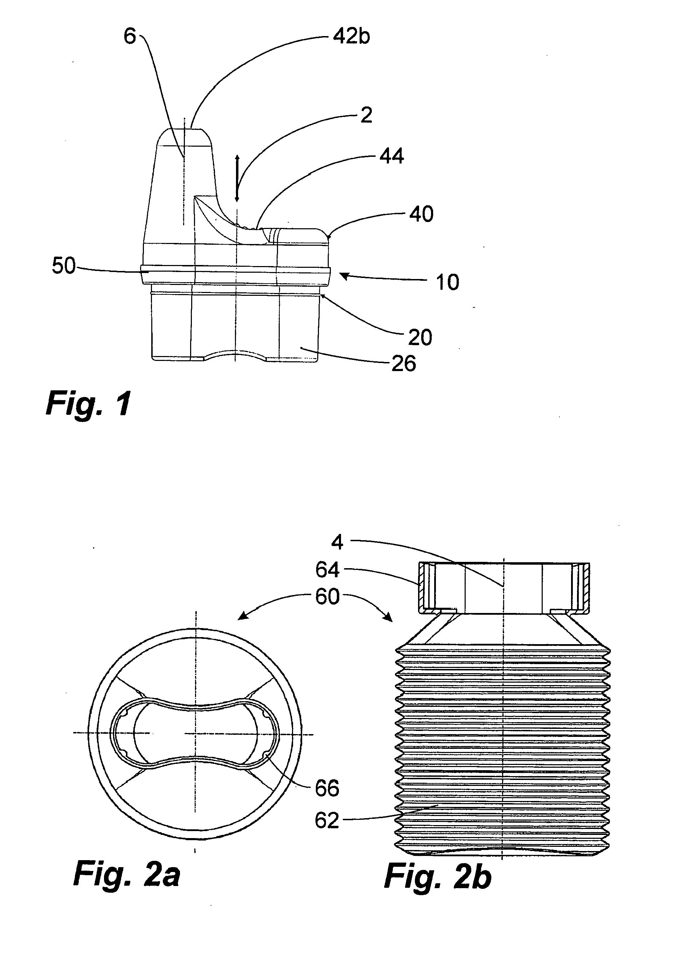

Figur 1 zeigt ein Gehäuse 10 einer erfindungsgemäßen Inhalationsvorrichtung. Dieses Gehäuse 10 besteht aus einem ersten Gehäuseteil 20, in dem in Figur 1 nicht sichtbarer Art und Weise zwei Medienspeicher 24 vorgesehen sind, die jeweils mit einem pulverförmigen Medium 30 gefüllt sind. In einem Kontaktbereich am unteren Ende ist das erste Gehäuseteil 20 als Konusstumpf 26 ausgebildet. Auf das erste Gehäuseteil 20 ist ein zweites Gehäuseteil 40 aufgesetzt, welches auf der dem ersten Gehäuseteil 20 gegenüberliegenden Seite einen Auslasskanal 42 sowie eine Fingerauflage 44 aufweist. Die beiden Gehäuseteile 20, 40 sind in dem dargestellten Zustand, der dem Lieferzustand des Gehäuses 10 entspricht, mittels eines gleichzeitig als Originalitätsschutz dienenden Sicherungsrings 50 miteinander dahingehend verbunden, dass sie in Richtung einer Hauptachse 2 des Gehäuses 10 vor Entfernen des Sicherungsrings 50 nicht relativ zueinander beweglich sind.

Die zweite Komponente der hier beschriebenen erfindungsgemäßen Inhalationsvorrichtung ist die Atemunterstützungsvorrichtung 60, die in den Figuren 2a und 2b in einer Vorderansicht und einer Draufsicht dargestellt ist. Sie weist einen Faltenbalg 62 auf, der eine Kompressionskammer 62 bildet. Am oberen Ende schließt sich ein zylindrischer Anschlussabschnitt 64 an den Faltenbalg 62 an. Die gesamte Atemunterstützungsvorrichtung 60 ist einstückig aus einem luftundurchlässigen und elastischen Material gefertigt, vorzugsweise aus Polyethylen. An der Innenseite des Anschlussabschnitts 64 sind insgesamt vier Spannrippen 66 vorgesehen, die als lokale Verdickungen des Anschlussabschnitts 64 ausgebildet sind.

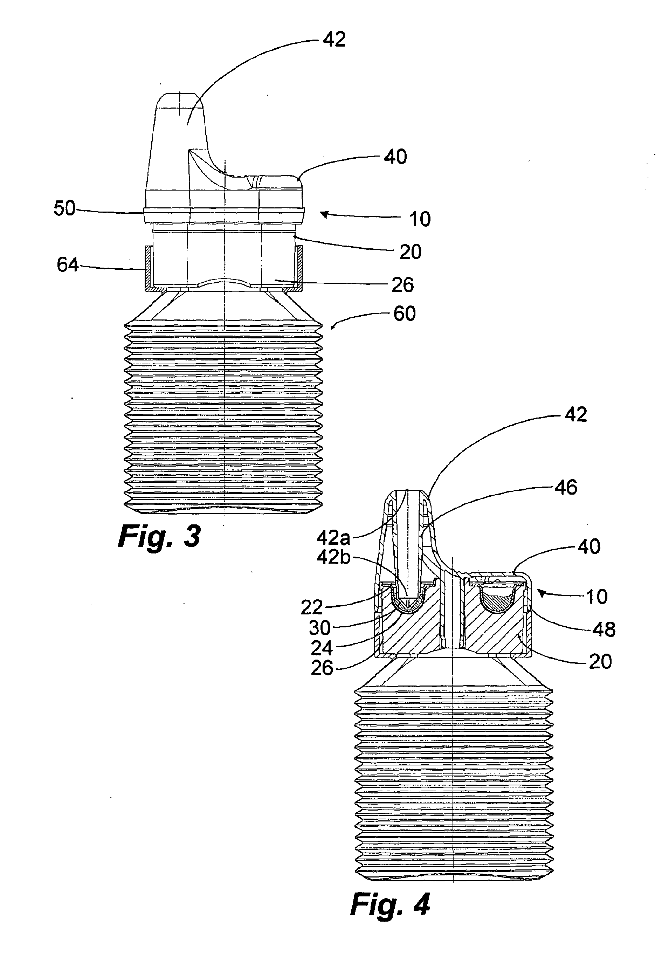

Figur 3 zeigt das Gehäuse 10 der Inhalationsvorrichtung sowie die Atmungsunterstützungsvorrichtung 60 in einem zusammengesetzten Zustand, wobei die Atmungsunterstützungsvorrichtung 60 im Bereich des Anschlussabschnitts 64 geschnitten dargestellt ist. Das Gehäuse 10 ist dabei immer noch in dem gleichen durch den Sicherungsabschnitt 50 gesicherten Zustand wie in Figur 1. Der elastisch ausgebildete Anschlussbereich 64 ist auf den konischen Kontaktbereich 26 des ersten Gehäuseteils 20 des Gehäuses 10 aufgeschoben. Während des Aufschiebens gewährleistet der Sicherungsabschnitt 50, dass die beiden Gehäuseteile 20, 40 sich nicht relativ zueinander bewegen. Der Konusstumpf 26 des ersten Gehäuseteils 20 und der Anschlussabschnitt 64 sind dabei so dimensioniert, dass der Anschlussabschnitt 64 tangential elastisch gespannt ist, so dass eine kraftschlüssige Verbindung zwischen der Atmungsunterstützungsvorrichtung 60 und dem Gehäuse 10 zustande kommt.

Figur 4 zeigt in einer teilweise geschnittenen Darstellung die Inhalationsvorrichtung nach Entfernen des Sicherungsrings 50 und nach der Aktivierung, die durch ein Ineinanderschieben der beiden Gehäuseteile 20, 40 herbeigeführt wird. Dabei wird im Zuge des Einschiebens des zweiten Gehäuseteils 40 in den ersten Gehäuseteil 20 ein innerer Kanalabschnitt 46 des Auslasskanals 42 durch eine Schutzfolie 22 hindurch in einen der beiden Medienspeicher 24 eingedrückt und dieser somit geöffnet. Das zweite Gehäuseteil 40 wird soweit auf das erste Gehäuseteil 20 aufgeschoben, bis ein ringförmiger Kontaktbereich 48 des zweiten Gehäuseteils 40 auf der ringförmigen Stirnfläche des Anschlussabschnitts 64 der Atmungsunterstützungsvorrichtung 60 aufliegt.

In diesem aktivierten Zustand wird das Inhalationsgerät vom Benutzer an seine Nase herangeführt und der Auslasskanal 42 in ein Nasenloch eingeführt, wobei die Außenwandung des Auslasskanals 42 bündig mit dem Nasenloch abschließt, und Einatmen durch dieses Nasenloch nur noch durch den Auslasskanal 42 hindurch möglich ist. Zur Inhalation des Mediums 30 verschließt der Benutzer dann mit der einen Hand das andere Nasenloch und betätigt dann mit der anderen Hand die Inhalationsvorrichtung bei gleichzeitigem Einatmen durch die Nase. Die Betätigung erfolgt durch Zusammendrücken des Faltenbalgs 62 in einer Betätigungsrichtung 4. Sie erfolgt, indem der Zeigefinger der betätigenden Hand auf der Fingerauflage 44 ruhend und der Daumen auf der Unterseite 68 des Faltenbalgs 62 ruhend zusammengedrückt werden. Durch die parallele Ausrichtung der Haupterstreckungsrichtung 6 des Auslasskanals 42 und der Betätigungsrichtung 4 des Faltenbalgs 62 und deren geringer Beabstandung ist ein Abrutschen aus dem Nasenloch nicht zu befürchten. Allenfalls eine leichte und unbedeutende Verschiebung der Vorrichtung in Haupterstreckungsrichtung 6 des Auslasskanals 42 kann Folge der Betätigung sein. Dies ist jedoch für eine erfolgreiche Inhalation unproblematisch.

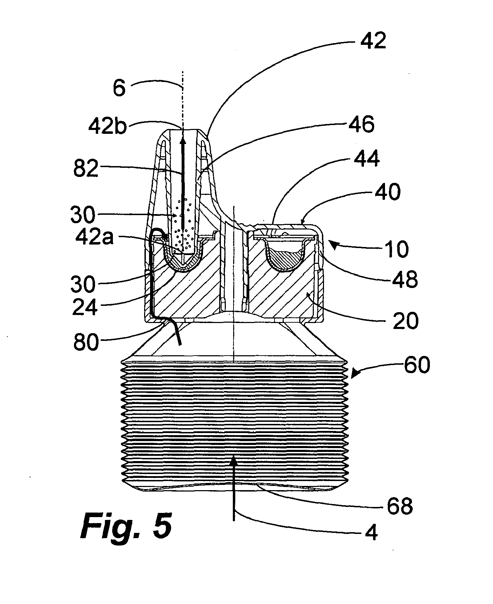

Wie in Fig. 5 dargestellt ist, entweicht die im Faltenbalg 62 zuvor befindliche Luft aus dem Faltenbalg 62 entlang der Pfeilrichtung 80 in das Gehäuse 10. Sie strömt in den Medienspeicher 24 und bläst das darin befindliche pulverförmige Medium 30 in die Eingangsseite 42a des Auslasskanals 42. Gleichzeitig wird die Luft durch die Ausgangsseite 42b hindurch durch den Einatemvorgang des Benutzers entlang der durch den Pfeil 82 dargestellten Strecke in die Nase des Benutzers gesogen. Der Überdruck, den die Luft aus dem Faltenbalg 62 im Gehäuse 10 verursacht, sowie der Unterdruck im Auslasskanal 42 durch das Einatmen seitens des Benutzers führen gemeinsam zu einer vollständigen Förderung des pulverförmigen Mediums 30 durch den inneren Kanalabschnitt 46 des Auslasskanals 42 hindurch in die Nase des Benutzers.

Selbst wenn der Auslasskanal 42 nicht bündig an der Innenseite des Nasenlochs des Benutzers anliegt sowie selbst wenn der Benutzer zu schwach oder kurzatmig ist, um während des gesamten Inhalationsvorgangs ausreichend kräftig einatmen zu können, wird durch den Überdruck im Gehäuse 10 dennoch eine vollständige Förderung des pulverförmigen Mediums erreicht.

Nachdem das pulverförmige Medium aus den Medienspeichern verbraucht wurde, kann der Faltenbalg vom Gehäuse wieder abgezogen werden und an ein neues, noch nicht verbrauchtes Gehäuse eines Inhalationsgerätes wieder angesteckt werden.

Das Gehäuse 10 der Inhalationsvorrichtung kann ohne konstruktive Änderungen auch ohne Faltenbalg 62 benutzt werden, so dass ein hohes Maß an Flexibilität erreicht wird: Benutzer, die mit der Inhalation Schwierigkeiten haben, verwenden das Inhalationsgerät mitsamt dem Faltenbalg 62, solche Benutzer, denen die Benutzung ohne Faltenbalg 62 keine Probleme bereitet, können auf dessen Verwendung verzichten.

Bei weiteren nicht dargestellten Ausführungsformen weisen die Gehäuse lediglich einen Medienspeicher oder mehr als zwei Medienspeicher auf.

Ebenfalls von der Erfindung umfasst sind solche nicht dargestellten Ausführungsformen, bei denen der Strömungsweg der Luft aus der Atmungsunterstützungsvorrichtung in das Gehäuse der Inhalationsvorrichtung nicht durch Nuten oder Spannrippen im Kontaktbereich zwischen den Komponenten verläuft, sondern als separater Lufteinlass vorzugsweise zentrisch an einer Unterseite des Gehäuses vorgesehen ist.

Auch andere Atmungsunterstützungsvorrichtungen, insbesondere mit elektrisch betriebenen Pumpen, können bei weiteren Ausführungsformen der erfindungsgemäßen Inhalationsvorrichtung verwendet werden.

## Patentansprüche

1. Inhalationsvorrichtung für ein insbesondere pulverförmiges Medium (30) mit einem Gehäuse (10) mit
- mindestens einem Medienspeicher (24) und
- einem Auslasskanal (42),
wobei ein erstes Ende (42a) des Auslasskanals (42) im Bereich des Medienspeichers (24) mündet und ein zweites Ende (42b) zum bündigen Ansetzen an eine Atemöffnung eines Benutzers ausgebildet ist, so dass das im Medienspeicher (24) gelagerte Medium (30) durch Einatmen seitens des Benutzers inhalierbar ist,
**gekennzeichnet durch**
eine Atmungsunterstützungsvorrichtung (60), die lösbar mit dem Gehäuse (10) verbindbar ist und aus der dem Gehäuse (10) ein die Inhalation unterstützender Luftstrom mittels einer manuellen Betätigung zuführbar ist.

2. Inhalationsvorrichtung gemäß Anspruch 1,
**dadurch gekennzeichnet, dass** die Atmungsunterstützungsvorrichtung (60) für eine manuelle Betätigung in einer Betätigungsrichtung (4) ausgebildet ist, die zu einer Haupterstreckungsrichtung (6) des Auslasskanals (42) parallel ist.

3. Inhalationsvorrichtung gemäß Anspruch 1 oder 2,
**dadurch gekennzeichnet, dass** die Atmungsunterstützungsvorrichtung (60) eine Kompressionskammer (62) aufweist, deren Volumen durch die manuelle Betätigung unmittelbar mechanisch reduzierbar ist.

4. Inhalationsvorrichtung gemäß Anspruch 3,
**dadurch gekennzeichnet, dass** die Atmungsunterstützungsvorrichtung (60) einen Faltenbalg (62) aufweist.

5. Inhalationsvorrichtung gemäß einem der vorangehenden Ansprüche,
**dadurch gekennzeichnet, dass** an einer der Atmungsunterstützungsvorrichtung (60) gegenüberliegenden Seite des Gehäuses (10) eine Fingerauflagefläche (44) vorgesehen ist.

6. Inhalationsvorrichtung gemäß einem der vorangehenden Ansprüche,
**dadurch gekennzeichnet, dass** die Atmungsunterstützungsvorrichtung (60) und das Gehäuse (10) in einem Kontaktbereich derart ausgebildet sind, dass eine durch die manuelle Betätigung aufgebrachte Kraft zu einem dichten Aufeinanderpressen von gehäuseseitigen und atmungsunterstützungsvorrichtungsseitigen Abschnitten (48, 64) im Kontaktbereich führt.

7. Inhalationsvorrichtung gemäß einem der vorstehenden Ansprüche,
**dadurch gekennzeichnet, dass**
die Atmungsunterstützungsvorrichtung (60) und das Gehäuse (10) in einem Kontaktbereich kraft- und/oder formschlüssig wirkende Dicht- und/oder Sicherungsmittel (26, 64, 66) aufweisen.

8. Inhalationsvorrichtung gemäß Anspruch 7,
**dadurch gekennzeichnet, dass**
die Sicherungsmittel einen gehäuseseitigen Aufschubkonus (26) und atmungsunterstützungsvorrichtungsseitigen Aufschubabschnitt (64, 66) aufweisen.

9. Inhalationsvorrichtung gemäß einem der vorstehenden Ansprüche,
**dadurch gekennzeichnet, dass**
das Gehäuse (10) mehrteilig, insbesondere zweiteilig ausgebildet ist, wobei in einem ersten Gehäuseteil (20) der mindestens eine Medienspeicher (24) ausgebildet ist und an einem zweiten Gehäuseteil (40) der Auslasskanal (42) ausgebildet ist, und dass ein umlaufender Kontaktbereich zwischen der Atmungsunterstützungsvorrichtung (60) und dem zweiten Gehäuseteil (40) als im Betrieb nach außen luftdichter Kontaktbereich ausgebildet ist.

10. Inhalationsvorrichtung gemäß Anspruch 8,
**dadurch gekennzeichnet, dass** lösbare Aktivierungssperrmittel (50) am Gehäuse (10) vorgesehen sind, die bei der Zusammenfügung des Gehäuses (10) mit der Atmungsunterstützungsvorrichtung (60) verhindern, dass sich das erste Gehäuseteil (20) und das zweite Gehäuseteil (40) relativ zueinander verschieben.

11. Inhalationsvorrichtung gemäß einem der vorstehenden Ansprüche,
**dadurch gekennzeichnet, dass**
das Gehäuse im Spritzgussverfahren und/oder der Faltenbalg im Spritzblasverfahren hergestellt ist.
